Europäisches Patentamt

European Patent Office

Office européen des brevets

(19)

(11) Veröffentlichungsnummer: **0 167 719**
**B1**

# (12) EUROPÄISCHE PATENTSCHRIFT

(45) Veröffentlichungstag der Patentschrift:
**20.04.88**

(51) Int. Cl.⁴: **A 61 B 17/56, A 61 B 17/16**

(21) Anmeldenummer: **85104200.2**

(22) Anmeldetag: **06.04.85**

(54) Zielgerät zur Herstellung von Querbohrungen in Knochen.

(30) Priorität: **08.06.84 DE 8417428 U**

(43) Veröffentlichungstag der Anmeldung:
**15.01.86 Patentblatt 86/3**

(45) Bekanntmachung des Hinweises auf die Patenterteilung:
**20.04.88 Patentblatt 88/16**

(84) Benannte Vertragsstaaten:
**AT CH DE FR GB IT LI SE**

(56) Entgegenhaltungen:
**AT - B - 365 914**
**DE - A - 3 245 680**
**DE - C - 734 245**
**US - A - 2 200 120**

(73) Patentinhaber: **Howmedica International, Inc.**
**Zweigniederlassung Kiel,**
**Professor-Küntscher-Strasse 1-5, D-2301 Schönkirchen üb. Kiel (DE)**

(72) Erfinder: **Olerud, Sven Emanuel, Prof., Box 4,**
**S-74011 Laennholm (SE)**
Erfinder: **Richter, Karl Manfred, Dipl.-Ing., Haferkamp 14,**
**D-2304 Wendtorf (DE)**

(74) Vertreter: **Dipl.-Ing. H. Hauck Dipl.-Phys. W. Schmitz**
**Dipl.-Ing. E. Graalfs Dipl.-Ing. W. Wehnert Dr.-Ing. W.**
**Döring, Neuer Wall 41, D-2000 Hamburg 36 (DE)**

**Beschreibung**

Die Erfindung bezieht sich auf ein Zielgerät zur Herstellung von Querbohrungen in Knochen in Übereinstimmung von Löchern oder Bohrungen eines Osteosynthesehilfsmittels in Knochen, insbesondere eines Verriegelungsnagels, mit einer Halterung zur Aufnahme eines in den Strahlengang eines Röntgengerätes bringbaren Zielglieds.

Verriegelungsnägel für die Versorgung von Knochenfrakturen, z.B. des Femurs oder der Tibia, werden mit Hilfe von Querschrauben im Knochen fixiert. Damit die Querschrauben durch die Querbohrungen im Verriegelungsnagel hindurchgeführt werden können, müssen ausgerichtete Querbohrungen im Knochen hergestellt werden. Diesem Zweck dient das Zielgerät.

Es ist ein Zielgerät bekanntgeworden, mit einem Zielkopf, der eine Bohrung zur Aufnahme einer Zielhülse aufweist und der an einer länglichen Zielkopfhalterung so angebracht ist, dass er in Längsrichtung der Halterung verstellbar ist (DE-GM 78 05 301). Die Zielkopfhalterung ist in einer Fassung angebracht, die mit einer Röntgenstrahlenquelle verbunden ist. Die Halterung ist der Fassung verschiebbar angeordnet. Das bekannte Zielgerät ist mit dem Röntgengerät verbunden und abhängig von diesem. Es ist daher auch bekanntgeworden, ein distales Zielgerät zu schaffen, das unabhängig vom Typ oder Farbikat des verwendeten Röntgengerätes ist (DE-GM 82 08 970.1). Bei dem bekannten Zielgerät ist eine Nagelhalterung zur lösbaren Aufnahme des proximalen Endes des Verriegelungsnagels annähernd parallel zur länglichen Zielkopfhalterung vorgesehen, die ihrerseits an einem Haltearm angebracht ist, die um die Achse des Verriegelungsnagels in der Nagelhalterung verschiebbar ist. Der Haltearm ist lösbar an der Zielkopfhalterung angebracht und in einer Richtung annähernd parallel zur Nagelachse in der Zielkopfhalterung verschiebbar. Zwei Justierstifte können senkrecht zur Verriegelungsnagelachse vom Zielkopf aufgenommen werden. Das bekannte Gerät zeichnet sich zwar durch relativ hohe Zielgenauigkeit aus, seine Handhabung erfordert jedoch viel Zeit. Ausserdem ist der apparative Aufwand beträchtlich.

Der Erfindung liegt die Aufgabe zugrunde, ein distales Zielgerät zu schaffen, das sich trotz hoher Zielgenauigkeit durch eine einfache und schnelle Handhabung auszeichnet.

Diese Aufgabe wird erfindungsgemäss dadurch gelöst, dass in der Halterung ein Aufnahmekopf aus einem für Röntgenstrahlung durchscheinenden Werkstoff drehbar gelagert ist zur Aufnahme eines Bohrers oder eines Bohrdrahtes, und an der Halterung ferner eine kraftgetriebene Antriebsmaschine angeordnet ist zum drehenden Antrieb des Aufnahmekopfes.

Das erfindungsgemässe Zielgerät dient nicht nur dazu, den Punkt ausfindig zu machen, an dem im Knochen die Querbohrung vorgenommen werden muss, sondern gleichzeitig dazu, eine Vorbohrung vorzunehmen. Da der Aufnahmekopf für einen Bohrer bzw. einen Bohrdraht für die Röntgenstrahlung durchscheinend ist, erscheint der Bohrer auf dem Bildschirm als Punkt. Dieser Punkt wird beim Zielen in Übereinstimmung mit einem Loch bzw. einer Querbohrung des Verriegelungsnagels gebracht. Ist dies geschehen, wird mit Hilfe des Bohrers bzw. des Bohrdrahtes durch die Weichteile und den Knochen gebohrt. Anschliessend wird das Zielgerät entfernt und über den Bohrdraht bzw. den Bohrer wird der Aufbohrvorgang mit Hilfe eines Hohlbohrers für die passende Knochenschraube vorgenommen. Anschliessend wird der Bohrdraht bzw. der erste Bohrer entfernt. Es versteht sich, dass bei Verwendung eines Bohrdrahtes bzw. eines geeigneten Bohrers in Verbindung mit dem Zielgerät der Durchmesser verhältnismässig gering ist, damit mit Hilfe des Hohlbohrers die Aufbohrung vorgenommen werden kann.

Das erfindungsgemässe Zielgerät zeichnet sich durch eine relativ hohe Zielgenauigkeit aus. Sie hat den Vorteil, dass noch während des Vorbohrens eine Kontrolle über einen Bildschirm durchgeführt werden kann.

Es wurde bereits erwähnt, dass nach dem Bohren mit Hilfe des Bohrdrahtes das Zielgerät vom Bohrdraht gelöst werden muss. Eine Ausgestaltung der Erfindung sieht hierzu vor, dass der Aufnahmekopf eine Kunststoffscheibe aufweist mit einer Öffnung zur lösbaren Aufnahme einer Spannhülse aus Kunststoff. Die drehbar gelagerte Kunststoffscheibe ist für Röntgenstrahlung durchscheinend. Das gleiche gilt für die lösbare Spannhülse, die den Bohrdraht aufnimmt. In diesem Zusammenhang sieht eine weitere Ausgestaltung der Erfindung vor, dass die Öffnung polygonal ist und die Spannhülse eine entsprechende Aussenkontur aufweist sowie einen sich gegen eine Seite der Kunststoffscheibe legenden Flansch. Die Öffnung ist z.B. quadratisch. Die Spannhülse hat eine entsprechende Aussenkontur und wird in die Öffnung eingesteckt, so dass bei einer Drehung der Kunststoffscheibe die Spannhülse mitgenommen wird. Die Spannhülse wird auf der dem Patienten zugewandten Seite der Kunststoffscheibe in die Öffnung eingesteckt, wobei sich während des Bohrvorgangs der Flansch gegen die Kunststoffscheibe anlegt, um ein Widerlager für den axialen Druck beim Bohren zu bilden.

Die Kunststoffscheibe wird während des Bohrvorgangs in Drehung versetzt. Es gibt verschiedene konstruktive Möglichkeiten eines geeigneten Antriebs für die Kunststoffscheibe. Eine besteht erfindungsgemäss darin, dass die Kunststoffscheibe am Umfang eine Zahnung aufweist. Die Zahnung steht mit einem geeigneten Ritzel in Eingriff, das über ein Winkelgetriebe mit der Welle der Antriebsmaschine in Wirkverbindung steht.

Es ist zwar denkbar, die Halterung für das Zielgerät in einem stationären Gerät verstellbar anzuordnen. Eine freie Handhabung der Halterung ist indessen vorzuziehen. Eine Ausgestaltung der Erfindung sieht hierzu vor, dass die Halterung ein längliches Blechteil aufweist und der Aufnahmekopf annähernd mittig in einem Lagergehäuse gelagert ist, das an einer Seite des Blechteils angebracht ist. Das Blechteil kann aus Aluminium bestehen und eine Länge von 50 bis 60 cm aufweisen. Eine derartige Länge reicht aus, um die Hände der Bedienungsperson ausserhalb des direkten Strahlngsbereichs der Röntgenquelle zu halten.

Das Blechteil hat nach einer weiteren Ausgestaltung der Neuerung eine Spannvorrichtung für das Gehäuse einer pneumatischen oder elektrischen Antriebsmaschine. Die Antriebsmaschine kann eine übliche pneumatisch oder elektrisch oder batteriegetriebene Antriebsmaschine in Pistolenform sein, die mit ihrem Gehäuse klemmend am Blechteil befestigt werden kann. Es versteht sich, dass auch ein Spezialmotor verwendet werden kann. Bei der pistolenartigen Antriebsmaschine kann der Pistolengriff zugleich die eine Handhabe an einem Ende des Blechteils bilden.

Die Erfindung wird nachfolgend anhand von Zeichnungen näher erläutert.

Fig. 1 zeigt in Seitenansicht ein Zielgerät nach der Erfindung.

Fig. 2 zeigt eine Draufsicht auf Zielgerät nach Fig. 1.

Fig. 3 zeigt einen Bohrdraht mit einer Spannhülse für ein Zielgerät nach Fig. 1.

Fig. 4 zeigt schematisch den Aufbau einer Röntgenanlage mit einem Zielgerät nach der Erfindung.

Das insgesamt mit 10 bezeichnete Zielgerät weist ein Halteblech 11 aus Aluminium mit einer Dicke von 5 mm auf, das an den Enden griffartig verbreitert ist. Die Länge des Blechteils 11 beträgt etwa 50 bis 60 cm. Am linken Ende weist es eine Grifföffnung 12 auf. Am rechten Ende sind zwei parallele Schenkel 13, 14 gebildet. Der obere Schenkel 13 weist einen zweiteiligen Spannklotz 15 auf mit Schrauben 16, welche die beiden Teile des Spannklotzes 15 gegeneinander spannen. Der dem Schenkel 13 nächstgelegene Teil des Spannklotzes 15 ist in geeigneter Weise befestigt. Der Spannklotz 15 dient zur Aufnahme des Gehäuses 17 einer pistolenartigen Antriebsmaschine 18. Die Antriebsmaschine 18 wird pneumatisch betrieben und besitzt am unteren Ende des Pistolengriffes 19 eine Tülle 20 zur Verbindung mit einem Druckluftschlausch. Eine Befestigungsschelle 21 ist mit dem unteren etwas längeren Schenkel 14 verbunden und spannt den die Tülle 20 aufweisenden Rohrstutzen 22 ein. Der Pistolengriff 19, der mit zwei Auslösern 23, 24 versehen ist, welche in die Öffnung zwischen den Schenkeln 13, 14 hineinstehen, bildet mithin zugleich eine Handhabe für das Blechteil 11.

Im mittleren schmaleren Bereich des Blechteils 11 ist ein Lagergehäuse 42 angebracht, und zwar zur gleichen Seite wie der Spannklotz 15. Im Lagergehäuse 42 ist eine Kunststoffscheibe 25 mit einem mittigen quadratischen Loch 26 axial fest, jedoch drehbar gelagert. Der Umfang der Kunststoffscheibe 25 ist mit einer Zahnung 27 versehen. Die Zahnung 27 steht in Eingriff mit einem nicht gezeigten Antriebsritzel eines im Lagergehäuse 42 angeordneten nicht gezeigten Winkelgetriebes. Die Eingangswelle 28 des Winkelgetriebes ist verbunden mit einem Wellenabschnitt 29, der mit der Spindel 30 der pneumatischen Antriebsmaschine 18 in Verbindung ist. Die Spindel 30 steht aus der Nase 31 des Gehäuses 17 vor.

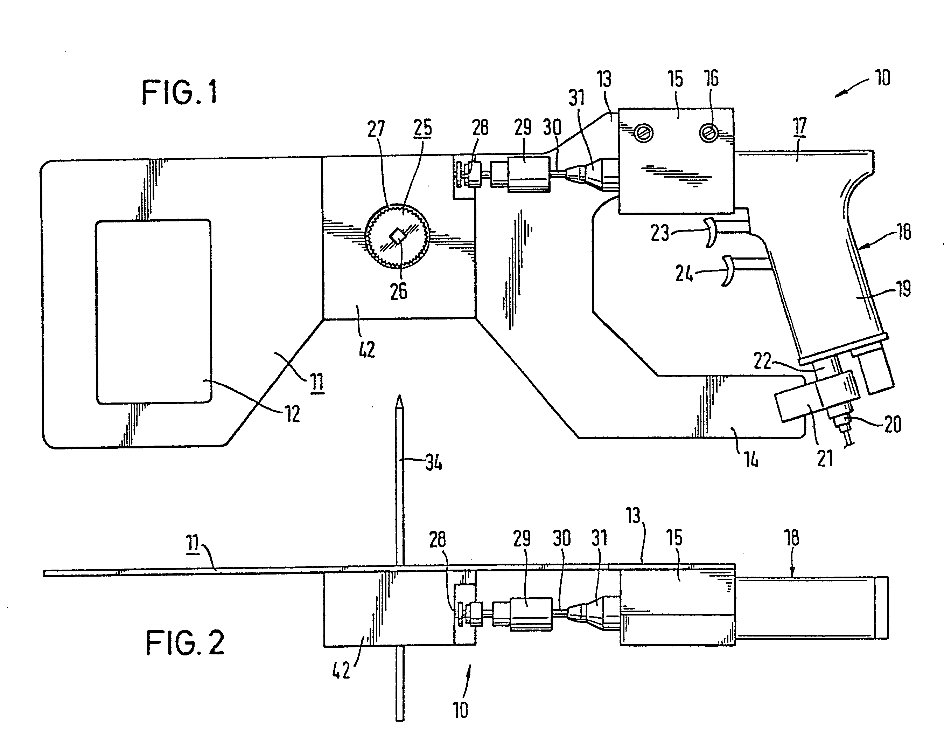

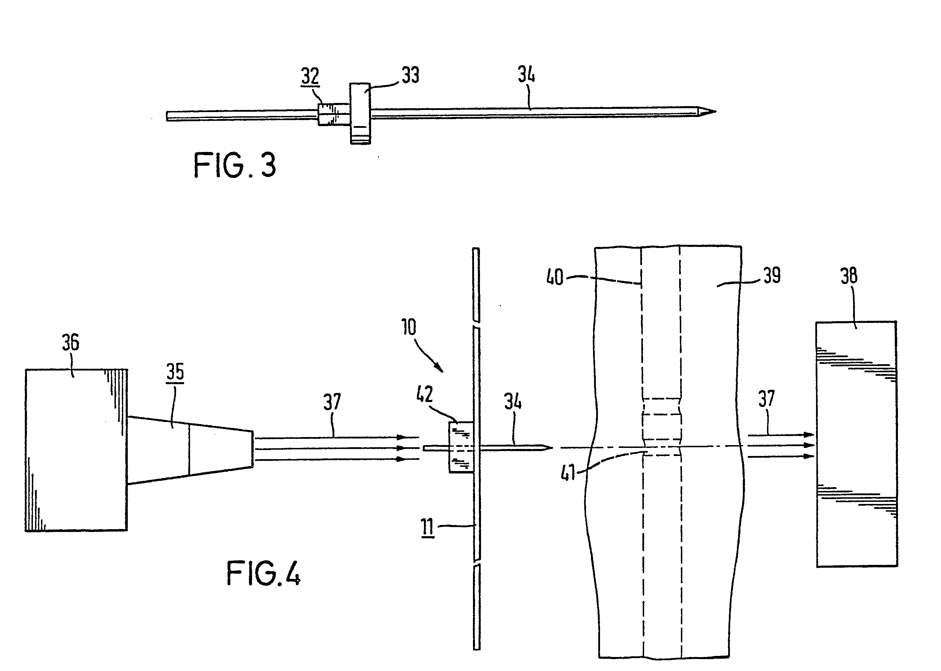

In Fig. 3 ist in Seitenansicht eine Spannhülse 32 von rechteckigem Querschnitt zu sehen, die an einem Ende einen Flansch 33 aufweist. Die Spannhülse 32 und Flansch 33 sind ebenfalls aus Kunststoffmaterial. Die Spannhülse 32 dient zur klemmenden Aufnahme eines Bohrdrahtes 34. Wie aus Fig. 2 zu erkennen ist, wird die Spannhülse 32 auf der dem Lagergehäuse 24 abgewandten Seite in die Öffnung 26 der Kunststoffscheibe 25 eingesteckt, wobei sich der Flansch 33 gegen die Aussenseite der Scheibe 25 anlegt.

In Fig. 4 ist schematisch eine Röntgenstrahlungsquelle 35 dargestellt, die an einer geeigneten Halterung 36 angeordnet ist. Im Strahlengang 37 der Röntgenquelle 35 befindet sich ein Empfangsgerät 38. Das Empfangsgerät ist mit einem nicht gezeigten Bildschirm verbunden. Im Strahlengang der Röntgenquelle 35 ist z.B. das Bein 39 eines Patienten angeordnet, in dem sich ein Verriegelungsnagel 40 (gestrichelt gezeichnet) befindet, der zuvor eingeschlagen wurde. Der Verriegelungsnagel 40 ist mit Querbohrungen 41 versehen. Wird das Zielgerät 10 im Strahlengang 37 gehalten, bildet sich der Bohrdraht 34 auf dem Bildschirm als Punkt ab. Er kann mithin durch Manipulieren durch Ergreifen des Blechteils 11 an beiden Enden in Übereinstimmung mit einer Querbohrung 41, die ebenfalls auf dem Bildschirm sichtbar wird, gebracht werden. Nach der Ausrichtung wird mit Hilfe des Bohrdrahtes 34 durch Weichteile und den Knochen eine Bohrung hergestellt. Nach Beendigung dieser Bohrung wird das Zielgerät 10 entfernt, wobei der Bohrdraht 34 im Bein verbleibt. Anschliessend wird um den Bohrdraht 34 mit Hilfe eines Hohlbohrers die endgültige Aufbohrung für eine Knochenschraube vorgenommen.

**Patentansprüche**

1. Zielgerät zur Herstellung von Querbohrungen im Knochen in Übereinstimmung mit Löchern oder Bohrungen eines Osteosynthesehilfsmittels im Knochen, insbesondere eines Verriegelungsnagels, mit einer Halterung (11) zur Aufnahme eines in den Strahlengang (37) eines Röntgengerätes (35) bringbaren Zielglieds, dadurch gekennzeichnet, dass in der Halterung (11) ein Aufnahmekopf (25) aus einem für Röntgenstrahlung durchscheinenden Werkstoff drehbar gelagert ist zur Aufnahme eines Bohreres oder eines Bohrdrahtes (34) und an der Halterung (11) ferner eine kraftgetriebene Antriebsmaschine (18) angeordnet ist zum drehenden Antrieb des Aufnahmekopfes (25).

2. Zielgerät nach Anspruch 1, dadurch gekennzeichnet, dass der Aufnahmekopf eine Kunststoffscheibe (25) aufweist mit einer Öffnung (26) zur lösbaren Aufnahme einer Spannhülse (32).

3. Zielgerät nach Anspruch 2, dadurch gekennzeichnet, dass die Öffnung (26) polygonal ist und die Spannhülse (32) eine entsprechende Aussenkontur aufweist sowie einen sich gegen eine Seite der Kunststoffscheibe (25) legenden Flansch (33).

4. Zielgerät nach Anspruch 2 oder 3, dadurch gekennzeichnet, dass die Kunststoffscheibe (25) am Umfang eine Zahnung (27) aufweist.

5. Zielgerät nach einem der Ansprüche 1 bis 4, dadurch gekennzeichnet, dass die Halterung ein längliches Blechteil (11) aufweist und der Aufnahmekopf annähernd mitting in einem Lagergehäuse (24) gelagert ist, das an einer Seite des Blechteils (11) angebracht ist.

6. Zielgerät nach Anspruch 5, dadurch gekennzeichnet, dass am Blechteil eine Spannvorrichtung (15) für das Gehäuse (18) einer pneumatischen oder elektrischen Antriebsmaschine (18) angeordnet ist.

7. Zielgerät nach Anspruch 5 oder 6, dadurch gekennzeichnet, dass das Lagergehäuse (24) ein Umlenkgetriebe enthält, das ein mit der Kunststoffscheibe (25) kämmendes Ritzel aufweist und das mit der Antriebswelle der Antriebsmaschine (18) in Wirkverbindung ist.

8. Zielgerät nach einem der Ansprüche 5 bis 7, dadurch gekennzeichnet, dass das Blechteil (11) an beiden Enden eine Handhabe aufweist.

## Claims

1. Aiming device for making transverse bores in the bone in registry with holes or bores of an osteosynthesis auxiliary means on the bone, in particular a locking nail, comprising a retention (11) for receiving an aiming element insertable in the beam path (37) of an X-ray device (35), characterized in that the retention (11) has rotatably supported therein a holding head (25) of a material pervious to X-radiation for receiving a drill or a drilling wire (34), and that the retention (11) has further arranged thereon a power-driven driving machine (18) for rotatably driving the holding head (25).

2. Aiming device as defined in claim 1, characterized in that the holding head comprises a plastic disc (25) with an aperture (26) for detachably receiving a clamping sleeve (32).

3. Aiming device as defined in claim 2, characterized in that the aperture (26) is polygonal, and that the clamping sleeve (32) has a correponding outer contour as well as a flange (33) engaging against one side of the plastic disc (25).

4. Aiming device as defined in claim 2 or 3, characterized in that the plastic disc (25) has a toothing (27) at its periphery.

5. Aiming device as defined in any of claims 1 to 4, characterized in that the retention includes an elongated sheet-metal member (11), and that the holding head is supported approximately centrally in a bearing housing (24) which is secured to one side of the sheet-metal member (11).

6. Aiming device as defined in claim 5, characterized in that the sheet-metal member has arranged thereon a clamping device (15) for the housing (18) of a pneumatic or electric driving machine (18).

7. Aiming device as defined in claim 5 or 6, characterized in that the bearing housing (24) in cludes a deflecting mechanism which includes a pinion meshing with the plastic disc (25) and is operatively connected with the drive shaft of the driving machine (18).

8. Aiming device as defined in any of claims 5 to 7, characterized in that the sheet-metal member (11) has a handle at both ends.

## Revendications

1. Appareil de visée pour l'exécution dans un os de trous transveraux en correpondance avec des trous d'un moyen d'ostéosynthèse, en particulier d'un clou de blocage, placé dans l'os, comportant un support (11) portant un organe de visée pouvant être mis sur le trajet des rayons (37) d'un appareil à rayons X (35), caractérisé en ce qu'une tête porte-outil (25), en matière laissant passer les rayons X, destinée à recevoir un foret ou une tige de perçage 634), est montée tournante dans le support (11), et une machine motrice (18) destinée à faire tourner ladite tête porte-outil (25) est en outre montée sur le support (11).

2. Appareil de visée selon la revendication 1, caractérisé en ce que la tête porte-outil comporte un disque en plastique (25) pourvu d'une ouverture (26) prévue pour recevoir une douille de serrage (32).

3. Appareil de visée selon la revendication 2, caractérisé en ce que l'ouverture (26) est polygonale et la douille de serrage (32) a un contour extérieur correspondant ainsi qu'un collet (33) qui s'applique contre une face du disque en plastique (25).

4. Appareil de visée selon l'une des revendications 2 et 3, caractérisé en ce que le disque en plastique (25) présente une denture (27) sur son pourtour.

5. Appareil de visée selon l'une des revendications 1 à 4, caractérisé en ce que le support comporte une plaque allongée (11) et la tête porte-outil est montée à peu près au milieu dans un corps de palier (24) qui est monté sur une face de la plaque (11).

6. Appareil de visée selon la revendication 5, caractérisé en ce que sur la plaque est monté un dispositif de serrage (15) destiné à serrer le carter (17) d'une machine motrice pneumatique ou életrique (18).

7. Appareil de visée selon l'une des revendications 5 et 6, caractérisé en ce que le corps de palier (24) contient un engrenage de renvoi qui présente un pignon en prise avec le disque en plastique (25) et est accouplé à l'arbre de sortie de la machine motrice (18).

8. Appareil de visée selon l'une des revendications 5 à 7, caractérisé en ce que la plaque (11) présente une poignée à chacune de ses deux extrémités.

FIG.1

FIG.2

0 167 719

FIG. 3

FIG. 4